# EUROPÄISCHE PATENTSCHRIFT

(11) **EP 0 636 620 B1**
(45) Veröffentlichungstag und Bekanntmachung des Hinweises auf die Patenterteilung: **15.10.1997**
(21) Anmeldenummer: 94110874.8
(22) Anmeldetag: 13.07.1994
(51) Int. Cl.: C07D 317/38

(54) **Verfahren zur Gewinnung von gereinigtem Ethylenglykolcarbonat (EGC)**
Method for obtaining purified ethyleneglycol carbonate
Procédé d'obtention d'éthylène glycol-carbonate purifié

(30) Priorität: 26.07.1993 DE 4325016
(43) Veröffentlichungstag der Anmeldung: 01.02.1995
(73) Patentinhaber: BAYER AG, 51368 Leverkusen (DE)
(72) Erfinder: Mendoza-Frohn, Dr. Christine, D-40699 Erkrath (DE); Wagner, Dr. Paul, D-40597 Düsseldorf (DE); Wirges, Dr. Hans-Peter, D-47800 Krefeld (DE)

(56) Entgegenhaltungen:
- GB-A- 1 086 028

## Beschreibung

Die vorliegende Erfindung betrifft ein Verfahren zur Gewinnung von gereinigtem Ethylenglykolcarbonat (EGC) aus einem verunreinigten EGC, welches Verunreinigungen aus der Gruppe von Ausgangsstoffen, Nebenprodukten und/oder Katalysatoren des Herstellungsverfahrens enthält, bei dem das verunreinigte EGC einer fraktionierenden Schmelzkristallisation unterworfen wird. Das hierbei gebildete Kristallisat von gereinigtem EGC wird anschließend mechanisch von den zurückbleibenden, in der Restschmelze gelösten Verunreinigungen abgetrennt. Diese neue Gewinnungsmethode ist energie- und materialsparend und benötigt keinen Einsatz von Zusatzstoffen, etwa von Lösungsmitteln. Dadurch werden besonders hohe Reinheitsgrade des EGC und die praktisch vollständige Wiederverwendung des Herstellungskatalysators ermöglicht.

Es ist bekannt, daß das EGC auf verschiedenen Wegen hergestellt werden kann, beispielsweise aus Ethylenglykol und Phosgen (DE-AS 12 26 117), aus Ethylenoxid bzw. Ethylenchlorhydrin und Kohlendioxid (Chem. Ing. Techn. 43 (1971), 903 ff.; Fette, Seifen, Anstrichmittel 73 (1971), 396 ff.; DE-OS 28 55 232, entsprechend US 4.314.945; Ind. Eng. Chem. 50 (1958), 767-770) sowie aus Ethylen, Sauerstoff und Kohlendioxid (US 3.025.305). Hierbei fällt das EGC in der Regel in einer durch die verschiedenen Stoffe aus dem Herstellungsprozeß verunreinigten Form an. Bei dem von Ethylenoxid und Kohlendioxid ausgehenden Verfahren enthält das Rohprodukt weiterhin den gelösten Katalysator, beispielsweise quartäre Ammoniumverbindungen.

Es sind daher eine Reihe von Reinigungsverfahren für das EGC bekannt geworden. Eine solche Reinigung erfolgt in den meisten Fällen durch Vakuumdestillation. Bei dieser Vakuumdestillation geht im Falke der Verunreinigung des EGC mit dem Ausgangsmaterial bzw. Nebenprodukt Glykol am Kolonnenkopf ein azeotropes Gemisch von Glykol und EGC über, während restliches EGC am Kolonnenfuß erhalten wird. Enthält das zu reinigende rohe EGC zusätzlich einen gelösten Katalysator aus dem Herstellungsverfahren, so muß dieser vor der beschriebenen Vakuumdestillation zur Entfernung von Glykol aus dem Rohprodukt abgetrennt werden. So werden beispielsweise bei dem in Chem. Ing. Techn. und Fette, Seifen, Anstrichmittel (loc. cit.) beschriebenen Verfahren zunächst zwei Dünnschichtdestillationen zur Abtrennung des Katalysators durchgeführt. Dieser abgetrennte Katalysator kann dann größtenteils wieder in die Herstellungsreaktion zum EGC zurückgeführt werden. In den Dünnschichtverdampfern werden gleichzeitig geringe Mengen an Nebenprodukten abgeschieden. Erst nach diesen Dünnschichtdestillationen wird die oben beschriebene Azeotropdestillation zur Entfernung von Glykol durchgeführt, und das am Kolonnenfuß erhaltene EGC wird einer weiteren Rektifizierung zugeführt. Durch diese Prozedur erzielt man, ausgehend von einem Rohprodukt mit 96-98 % EGC laut gaschromatographischer Analyse, ein Reinprodukt mit 99,5 % EGC, weiterhin mit 0,025 % Wasser und 0,1 % Glykol (wiederum laut gaschromatographischer Analyse).

Destillationsverfahren zur Reinigung von EGC besitzen im allgemeinen den Nachteil, daß eine Vakuumdestillationsanlage bei Drücken von maximal 50 mbar betrieben werden muß, da das EGC bei höheren Drücken, die gleichzeitig höheren Destillationstemperaturen entsprechen, bereits teilweise zersetzt wird und damit eine Verringerung der Ausbeute in Kauf genommen werden muß. Diese Gefahr der Zersetzung des EGC besteht insbesondere dann, wenn das Rohprodukt noch den Katalysator aus der Umsetzung, beispielsweise von Ethylenoxid mit Kohlendioxid, in gelöster Form enthält und dieser vor der destillativen Feinreinigung ebenfalls destillativ abgetrennt wird. So ist beispielsweise in Ind. Eng. Chem. (loc. cit.) beschrieben, daß der genannte Druck von maximal 50 mbar empfehlenswert ist, um eine gute Produktqualität zu gewährleisten. Da jedoch der Katalysator durch das Abdestillieren des reinen EGC im Destillationssumpf aufkonzentriert wird und andererseits der Grad der Zersetzung des EGC stark von der Konzentration des in ihm gelösten Katalysators abhängt, darf laut genannter Publikation bei einer Eingangskonzentration von 0,25 bis 0,5 Gew.-% Katalysator nicht mehr als 90 bis 95 % des EGC abdestilliert werden. Eine weitere Gefahr besteht darin, daß der Katalysator selber bei diesem Destillationsvorgang zersetzt werden kann. Will man also den Destillationssumpf, der eine konzentrierte Katalysatorlösung darstellt, in die Herstellungsreaktion zurückführen, muß ein Anteil des Katalysators verworfen und durch neuen Katalysator ersetzt werden. In der genannten Publikation wird beispielsweise ein Ersatz von 30 % der Katalysatorlösung empfohlen.

Es ist weiterhin bekannt, EGC durch Extraktion mit Lösungsmitteln zu reinigen. Hierbei kann ein Lösungsmittelgemisch, ein einziges Lösungsmittel, wie Ethylendichlorid, Methylendichlorid oder Chloroform, oder ein Kohlenwasserstoff gemeinsam mit Wasser (zur Aufnahme des wasserlöslichen Katalysators) zur Anwendung kommen (US 2.766.258). Dabei wird ein Reinheitsgrad von 99,1 % EGC erreicht. Der Nachteil eines Extraktionsverfahrens besteht in der notwendigen, sich anschließenden destillativen Trennung des EGC vom Extraktionslösungsmittel. Im Falle katalysatorbeladener Rohprodukte besteht bei der Extraktion zusätzlich die Gefahr, daß Reste der zur Extraktion benutzten Lösungsmittel bei der Katalysatorrückführung in den Reaktionskreislauf gelangen, wo sie zu Nebenprodukten und Zersetzungsreaktionen führen können.

Es ist auch bereits versucht worden, EGC durch Umkristallisation aus einem Lösungsmittel, beispielsweise aus Toluol, zu reinigen (US 2.994.705). Der Nachteil eines solchen Verfahrens besteht darin, daß zur Erreichung einer genügenden Reinheit eine mehrfache Umkristallisation erforderlich ist. Ein weiterer Nachteil, der in entsprechender Weise wie bei der Extraktion deutlich wird, besteht in der Notwendigkeit der Trennung eines etwa vorhandenen Katalysators vom Lösungsmittel der Umkristallisation.

In einer noch weiteren Methode zur Reinigung von rohem EGC wird dieses in kontinuierlicher Weise im Gegenstrom aus Ethylenglykol als Lösungsmittel kristallisiert (DE-AS 12 72 932). Hierbei wird eine vorgewärmte EGC-Lösung im oberen Teil einer Kristallisationskolonne bis zur Bildung von EGC-Kristallen abgekühlt, während man den unteren Teil der Kolonne bei einer Temperatur von 40 bis 50°C hält. Die Erwärmung und Kühlung werden so aufeinander abgestimmt, daß ein abwärts gerichteter Kristallfluß entsteht; das gereinigte EGC wird im geschmolzenen Zustand am Kolonnenboden abgezogen, und die erschöpfte Mutterlauge wird am oberen Teil der Kolonne abgenommen. Der kristalline Zustand des EGC ist in diesem Reinigungsverfahren nur ein kurzzeitig auftretender Zwischenschritt. Mit diesem Verfahren werden Reinheiten von 99,67 % EGC (laut gaschromatographischer Analyse) gewonnen, wenn man von einem Reaktionsgemisch ausgeht, das bei der Herstellung von EGC aus Ethylenglykol und Phosgen anfällt. In diesen Reaktionsgemischen liegen neben EGC und Glykol weiterhin Ethylenchlorhydrin und Salzsäure vor. Auf diese Anteile im Rohprodukt wird in DE-AS' 932 selbst nicht eingegangen; jedoch kann man diese aus Rev. Chim. (Bukarest) 17 (1966), 482-485 entnehmen, wo das in DE-AS' 932 beschriebene Verfahren näher ausgeführt wird. Setzt man demnach Gemische aus 50 bis 60 % EGC, 30 bis 40 % Ethylenglykol, 1 % Salzsäure, 4 bis 5 % Ethylenchlorhydrin und 5 % Wasser ein, fällt ein gereinigtes EGC mit folgenden Werten an: 96,4-98,3 % EGC, 0,65-1,41 % Ethylenglykol, 0,14-0,28 % Ethylenchlorhydrin und 0,19-0,39 % Wasser. Damit liegt jedoch ein gereinigtes EGC vor, dessen sehr hohe Reste an Ethylenglykol, Ethylenchlorhydrin und insbesondere Wasser bei der weiteren Verwendung des EGC zu dessen Zersetzung und zu Nebenreaktionen führen können.

Bei einer Gegenstromkristallisation aus Ethylenglykol wie der beschriebenen läge im Falle von katalysatorbeladenem rohem EGC der Katalysator nach der Kristallisation gemeinsam mit den Nebenkomponenten im Ethylenglykol gelöst vor. Möchte man den Katalysator in die Herstellungsreaktion zurückführen, müßte ein Trennungsschritt des Katalysators vom Ethylenglykol erfolgen, da das Ethylenglykol in Gegenwart der zum Einsatz gelangenden Katalysatoren und der dabei einzuhaltenden Betriebsbedingungen zu Nebenreaktionen des Ethylenoxids führen würde. Dies stellt eine äußerst nachteilige Belastung einer solchen Kristallisation dar.

Es war daher wünschenswert, ein Reinigungsverfahren für verunreinigtes EGC zur Verfügung zu haben, das energie- und materialsparend ist, das weder zur Zersetzung des EGC selbst noch des Katalysators führt und das darüber hinaus ohne Zusatzstoffe, wie Lösungsmittel für Extraktion oder Kristallisation, zu betreiben ist.

Es wurde nun überraschend gefunden, daß bei der fraktionierenden Kristallisation von EGC aus der Schmelze, also ohne Zusatz von Lösungsmitteln, hohe Reinheiten des EGC erhalten werden. Sowohl die Verunreinigungen aus dem rohen EGC als auch evtl. aus dem Herstellungsprozeß stammende und im rohen EGC gelöste Katalysatoren werden nur in einem äußerst geringen Ausmaß in das Kristallisat eingebaut.

Ein weiterer vorteilhafter Befund der erfindungsgemäßen Schmelzkristallisation ist die dabei besonders schonende Behandlung des Katalysators, der in der Schmelze verbleibt und daher im allgemeinen vollständig in die Reaktion zur Herstellung des EGC zurückgeführt werden kann. Ein weiterer Vorteil besteht in der hohen Kristallisationstemperatur von etwa 37°C und der damit verbundenen Möglichkeit, die Kristallisationswärme selbst bei sommerlichen Temperaturen durch zur Verfügung stehende Sekundärkühlkreislaufwässer auch bei einer Vorlauftemperatur von 29-30°C billig abführen zu können. Vorteilhaft ist ferner gegenüber Destillationsverfahren die Tatsache, daß die Kristallisationswärme des EGC nur etwa ein Drittel seiner Verdampfungswärme beträgt.

Es wurde ein Verfahren zur Gewinnung von gereinigtem Ethylenglykolcarbonat (EGC) aus einem verunreinigten EGC, das Verunreinigungen aus der Gruppe von Ausgangsstoffen, Nebenprodukten und/oder Katalysatoren des Herstellungsverfahrens enthält, gefunden, das dadurch gekennzeichnet ist, daß das verunreinigte EGC einer fraktionierenden Schmelzkristallisation unterworfen wird und das dabei gebildete Kristallisat des gereinigten EGC mechanisch von den zurückbleibenden, in der Restschmelze gelösten Verunreinigungen abgetrennt wird.

Als erfindungsgemäß einzusetzendes verunreinigtes EGC kommt sowohl ein vorgereinigtes EGC in Frage, das einer weiteren Feinreinigungsstufe unterzogen werden soll, als auch ein rohes Reaktionsgemisch, das direkt aus der Herstellung von EGC stammt. Wegen der Einfachheit des erfindungsgemäßen Verfahrens wird man in vielen Fällen darauf verzichten, EGC zunächst einer anders gearteten Reinigung zu unterziehen, um sodann das erfindungsgemäße Reinigungsverfahren anzuwenden; es ist daher bevorzugt, als verunreinigtes EGC ein Reaktionsgemisch aus der Herstellung von EGC direkt in erfindungsgemäßer Weise zu behandeln. Wegen der oben beschriebenen schonenden Entfernung eines Katalysators ist es weiterhin bevorzugt, solche Herstellungsgemische von EGC erfindungsgemäß einzusetzen, die einem katalysierten Herstellungsverfahren entstammen.

Das erfindungsgemäße Verfahren der fraktionierenden Schmelzkristallisation von verunreinigtem EGC kann sowohl diskontinuierlich als auch kontinuierlich durchgeführt werden, bevorzugt wird es diskontinuierlich durchgeführt.

Für die erfindungsgemäße Reinigung von EGC oder die Abtrennung von EGC aus gegebenenfalls katalysatorhaltigen Rohprodukten können Kristallisationsverfahren eingesetzt werden, wie sie beispielsweise in Chem. Ing. Techn. 57 (1985), 91; Chem. Ing. Techn. 63 (1991), 881 oder im Prospekt zur fraktionierten Kristallisation der Firma Sulzer vom August 1992 beschrieben sind. Hierbei handelt es sich beispielsweise um Verfahren mit Bildung zusammenhängender Kristallschichten, die häufig taktweise betrieben werden, wobei Rohrbündel-Kristallisatoren oder modifizierte Plattenwärmeaustauscher unterschiedlicher Konstruktion mit oder ohne Umpumpen der Schmelze, mit und ohne Anwendung von Pulsationen bzw. mit und ohne Unterteilung der Rohre in Segmente mit separater Ausspeisung zur Anwendung gelangen. Weiterhin kommen Fallfilmkristallisatoren unterschiedlicher Bauweise mit und ohne Einbauten zur Verbesserung des Wärmeübergangs in Frage, wie sie beispielsweise in EP 218 545 beschrieben werden. Weitere einsetzbare Apparate sind Blasensäulenkristallisatoren, Kristallisierwalzen und -bänder.

Eine weitere, kontinuierlich arbeitende Kristallisationseinrichtung mit Bildung zusammenhängender Kristallschichten mit einer umlaufenden Transporteinrichtung, mit Einrichtungen zum Zuführen der Schmelze und zum Abführen des gereinigten Produktes sowie Einrichtungen zum Schwitzen des Kristallisats ist in EP 521 499 beschrieben.

Zu den dem Fachmann bekannten Verfahren zur Schmelzkristallisation zählen weiterhin die Verfahren mit Bildung von Kristallsuspensionen, die in einem weiteren Verfahrensschritt, meist durch eine mechanische Flüssigabtrennung, in Feststoff und Restschmelze getrennt werden. Diese überwiegend kontinuierlich betriebenen Verfahren werden beispielsweise ausgeführt in Rührkessel-Kristallisatoren mit und ohne äußeren Umlauf, in Kratz- bzw. Schabkühlern unterschiedldicher Konstruktion mit oder ohne Kaskadierung, in Scheiben-Kristallisatoren bzw. statischen Kristallisatoren verschiedener Ausführung und in Kristallisierkolonnen, die entweder als Druckkolonnen, bei denen der Transport von Kristallen und Schmelze durch Pumpen oder Stempelpressen von außen bewirkt wird, oder als Kolonne mit mechanischen Zwangsfördereinrichtungen, wie speziellen Rührern, Schnecken, Wendeln oder Spiralen ausgeführt sind. Diese Kristallisierkolonnen können auch in Gegenstromfahrweise betrieben werden.

Bevorzugt werden für das erfindungsgemäße Verfahren Rohrbündel-Kristallisatoren, Plattenwärmeaustauscher unterschiedlicher Konstruktion mit und ohne Umpumpen der Schmelze oder Fallfilmkristallisatoren herangezogen.

Der Kristallisationsvorgang im erfindungsgemäßen Verfahren zur Reinigung eines bereits vorgereinigten EGC oder zur Abtrennung von EGC aus der gegebenenfalls katalysatorhaltigen Reaktionslösung aus der Herstellung kann sowohl durch spontane Keimbildung als auch durch eine definierte Zufuhr von Kristallisationskeimen (Impfung) eingeleitet werden. Beiden Formen sind in ihrer grundsätzlichen Anwendbarkeit bekannt. Wegen der stärker ausgeprägten Reproduzierbarkeit ist die Einleitung der Kristallbildung durch Kristallkeime bevorzugt.

Sowohl Arbeitsweisen mit Bildung zusammenhängender Kristallschichten als auch solche mit Bildung von Kristallsuspensionen, die beide im erfindungsgemäßen Verfahren durchgeführt werden können, können mehrstufig betrieben werden, um die erzielte Produktreinheit oder die geforderte Ausbeute an reinem Produkt zu erreichen. Dies kann beispielsweise erforderlich sein, wenn man von einem besonders verunreinigten Roh-EGC ausgeht und/oder besonders hohe Reinheitsanforderungen an das Rein-EGC stellt. Die einzelnen Stufen können in mehreren aufeinanderfolgenden Anlagekomponenten oder zeitlich verschoben im gleichen Kristallisator durchgeführt werden.

Das erfindungsgemäße Verfahren zur Gewinnung von gereinigtem EGC durch Schmelzkristallisation kann mit destillativen Reinigungsverfahren verbunden werden. So ist es möglich, vor Anwendung der Schmelzkristallisation, insbesondere bei der Abtrennung von EGC aus gegebenenfalls katalysatorhaltigen Reaktionslösungen, eine schonende Destillation leicht flüchtiger Reaktionsprodukte vorab vorzunehmen und erst dann den verbleibenden Destillationssumpf der erfindungsgemäßen Schmelzkristallisation zu unterwerfen. Eine solche schonende Vorabdestillation, beispielsweise unter vermindertem Druck und unter Anlegung einer vorgewählten Höchsttemperatur, steht im Einklang mit der produktschonenden erfindungsgemäßen Schmelzkristallisation.

Die Kombination des erfindungsgemäßen Verfahrens mit destillativen Trennschichten führt ebenfalls zu hohen Reinheitsgraden des dabei gewonnenen EGC sowie zu einem nur geringen Zersetzungsgrad von EGC und Katalysator während eines solchen kombinierten Reinigungs- bzw. Trennschrittes. Beispielsweise kann aus einem katalysatorhaltigen Rohprodukt zunächst in einer wahlweise mehrstufigen Kristallisation aus der Schmelze der Katalysator abgetrennt werden, wobei je nach Art des gewählten Verfahrens zur Schmelzkristallisation, je nach Wahl der anzuwendenden Parameter, wie Temperaturen, Abkühl- und Heizraten usw. und je nach Anzahl der Verfahrensstufen ein geringer Rest an Katalysator in dem Kristallisat verbleiben. Diese Spuren an Katalysator führen in einer anschließenden, schonenden Destillation im Vakuum nur noch in weitaus geringerem Maße zu Zersetzungen des EGC, als dies bei der Destillation des Rohprodukts in Gegenwart der gesamten Katalysatormenge der Fall ist. Ein zusätzlicher Vorteil einer solchen kombinierten Verfahrensführung ergibt gegenüber rein destillativen Verfahren eine praktisch vollständige Rückführbarkeit des Katalysators in den Reaktor zur EGC-Herstellung, die lediglich durch Bedingungen des EGC-Herstellungsprozesses begrenzt sein kann.

Reines EGC hat einen Schmelzpunkt von 36,4°C. Der sich hieraus zwangsläufig ergebende Temperaturbereich für das Verfahren überstreicht das Gebiet von 45°C bis hinunter zu 0°C.

Im Falle der Ausführung des erfindungsgemäßen Verfahrens in Rohrbündel-Kristallisatoren oder Plattenwärmeaustauschern wird die zu reinigende Schmelze im Bereich von 38-20°C, bevorzugt 37-25°C mit einer Kühlrate des Kühlmediums von 40-0,1 K/h, bevorzugt 10-0,5 K/h abgekühlt; während dieser Abkühlphase wird der Kristallisationsvorgang durch spontane Keimbildung oder durch definierte Keimzufuhr (Impfung), bevorzugt durch Impfung, eingeleitet. Bei der niedrigsten Temperatur des Kühlmediums kann vor der Abtrennung der Restschmelze eine Haltezeit von bis zu 100 Minuten eingehalten werden; ggf. kann diese Haltezeit entfallen. Die Haltezeit beträgt daher unter Berücksichtigung beider Varianten 0 bis 100 Minuten, bevorzugt 1 bis 70 Minuten. Danach erfolgt die Abtrennung der Restschmelze, und das Kristallisat wird durch sogenanntes "Schwitzen" mit einer Heizrate des Heizmediums von 40 bis 0,1 K/h, bevorzugt 20 bis 0,5 K/h, bis zu einer Endtemperatur von 22 bis 37°C, bevorzugt 28 bis 36°C weiter gereinigt.

Die für statische Kristallisationsverfahren unter Anwendung von Rohrbündeln oder von Plattenwärmeaustauschern beschriebenen Zusammenhänge gelten in grundsätzlich gleicher Weise auch für die Durchführung in Fallfilm-Kristallisatoren; jedoch müssen darüber hinaus noch weitere Zusammenhänge, wie beispielsweise die spezifische Rohrbelastung durch die Schmelze und der örtlichen Temperaturdifferenz zwischen Schmelze und Wärmemedium, in ihrem Einfluß auf die Reinheit des Kristallisats berücksichtigt werden. Diese Zusammenhänge sind dem Fachmann jedoch grundsätzlich bekannt.

Die im erfindungsgemäßen Verfahren einsetzbaren EGC-Rohprodukte können die Anteile an Verunreinigungen, Nebenprodukten, restlichen Ausgangsstoffen und Katalysatoren enthalten, die dem Fachmann aus den verschiedenen Herstellungsverfahren für EGC bekannt sind. Die bereits genannte Möglichkeit der mehrstufigen Ausführung des erfindungsgemäßen Verfahrens kann verschieden hohe Mengen an den genannten Verunreinigungen berücksichtigen. Als Beispiele für vorhandene Verunreinigungen können beispielsweise aus dem Herstellungsverfahren des EGC aus Ethylenoxid und CO₂ in Gegenwart von Katalysatoren genannt werden: die Katalysatoren; Produkte aus der Umlagerung des Ethylenoxids zu Acetaldehyd und dessen Polykondensation, wie lineare und cyclische, gegebenenfalls mehrfach ungesättigte Aldehyde; Glykol und lineare bzw. cyclische Produkte aus dessen Kondensation; Produkte aus der Reaktion von Ethylenoxid mit Glykol oder Acetaldehyd unter dem Einfluß des gegenwärtigen Katalysators.

Als Katalysatoren aus dem Herstellungsverfahren von EGC, die im Rahmen des erfindungsgemäßen Verfahrens als Verunreinigung entfernt werden können, kommen praktisch alle bisher vorgeschlagenen Katalysatoren in Frage. Dies sind beispielsweise: (Erd)alkalibromide und -iodide, Guanidine und deren Hydrobromide oder Hydroiodide, Tetraalkylammoniumbromide und -iodide, Phosphoniumbromide und -iodide, Pyridiniumhalogenide, Sulfonium-, Stibonium- und Arsoniumhalogenide, Zink- und Bleihalogenide, Alkylzinnverbindungen oder Gemische aus Alkalihalogeniden mit Halogeniden zweiwertiger Metallionen. Diese Katalysatoren sind dem Fachmann aus zahlreichen Veröffentlichungen bekannt. In besonderer Weise sei auf folgende Katalysatoren, die als zu entfernende Verunreinigungen vorhanden sein können, hingewiesen: Alkalibromide und -iodide, Tetraalkylammoniumbromide und -iodide, Phosphoniumhalogenide, Guanidiniumhalogenide und Gemische aus Alkalihalogeniden mit Halogeniden zweiwertiger Metalle.

### Beispiele 1 bis 8

Die in den Tabellen 1 und 2 aufgeführten Daten für die Beispiele 1 bis 6 zur Reinigung von EGC und/oder zur Abtrennung von EGC aus katalysatorhaltigen Rohprodukten wurden in einem Rohrbündel-Kristallisator (Rohrdurchmesser 4 cm) gewonnen, die Daten für die Beispiele 7, 8 in einem Fallfilmkristallisator (Rohrdurchmesser 4,9 cm; Höhe 1,2 m). Die Beispiele 1-6 wurden mit definierter Keimzufuhr (Impfung) durchgeführt.

Tabelle 1 enthält für die sechs im Rohrbündel-Kristallisator durchgeführten Beispiele die Werte für die Start- und Endtemperatur der Abkühlphase, für die Impftemperatur, Kühlrate des Kühlmediums, Haltezeit, Heizrate des Heizmediums und den Grad der Abtrennung Kristallisat/Schmelze. Im Fallfilmkristallisator wurde bei konstanter Kristallisationstemperatur gearbeitet; eine Impfung entfiel. Die Dosiergeschwindigkeit des zu reinigenden Eduktes betrug in den Beispielen 7 und 8 30 g/min.

Die Analysenergebnisse zu den acht Beispielen sind in Tabelle 2 aufgeführt.

Die Zusammensetzungen des Rohproduktes, des Kristallisats und der Restschmelze wurden mit Hilfe eines Gaschromatographen ermittelt. Als weiteres, sehr empfindliches Maß für die Reinheit von Rohprodukt, Kristallisat und Schmelze kann die Farbzahl nach Hazen angesehen werden, die den auf die polykondensierten Aldehyde zurückzuführenden Gelbstich in den EGC-Proben quantifiziert. Die Katalysatorkonzentrationen in Rohprodukt, Kristallisat und Schmelze wurden im Falle der anorganischen Salze mit Hilfe der Atom-Absorptions-Spektroskopie, im Falle der Ammoniumhalogenide über Analyse des Halogenids durch argentometrische Titration ermittelt.

Die Beispiele 1 bis 6 belegen, daß schon mit einer einstufigen Rohrbündel-Kristallisation hohe Reinheiten des EGC und niedrige Einbauraten des Katalysators in das Kristallisat erreicht werden können. Im Falle der EGC-Abtrennung aus katalysatorhaltigen Rohprodukten, bei denen die katalysatorhaltige Schmelze in den Herstellungsprozeß zurückgeführt wird, werden auch diese Schmelzen aufgrund der schonenden Trennmethode nicht zusätzlich thermisch belastet und weisen daher ebenfalls eine hohe Reinheit auf.

Die Beispiele 1 und 2 belegen den Einfluß der Konzentration an Verunreinigungen im Roh-EGC auf die Qualität des Rein-EGC: Verunreinigungen, wie Glykol und polykondensierte Aldehyde wurden kaum in das Kristallisat eingebaut und sammelten sich bevorzugt in der Schmelze. Trotz der sehr unterschiedlichen Farbzahlen der Rohprodukte in beiden Beispielen wiesen die Kristallisate in beiden Fällen eine Farbzahl von 10 (farblos) auf. Beim Glykol erkannte man einen etwas erhöhten Einbau in das Kristallisat bei höherer Konzentration im Rohprodukt (Beispiel 1).

Beispiel 4 zeigt im Vergleich zu Beispiel 3, daß eine höhere Katalysatorkonzentration im Rohprodukt bei sonst identischen Bedingungen zu einer leicht höheren Einbaurate des Katalysators in das Kristallisat führt. Die Katalysatorkonzentration im Kristallisat war mit 0,03 bis 0,04 % für eine einstufige Kristallisation sehr gering. Die Einbaurate des Katalysators ist weiterhin abhängig von der Art des Katalysators, wie der Vergleich der Beispiele 4, 5 und 6 belegt. Bei allen Katalysatoren konnte aber eine deutliche Aufkonzentrierung in der Schmelze registriert werden.

Beispiele 7 und 8 zeigen, daß durch die Ausführung der EGC-Reinigung in einem Fallfilmkristallisator bei gleichem Grad der Abtrennung Kristallisat/Schmelze noch höhere Abtrennraten an Katalysator und Nebenkomponenten erreicht werden können als bei der statischen Ausführung im Rohrbündelkristallisator, d.h. eine höhere Qualität des Rein-EGC erreichbar ist..

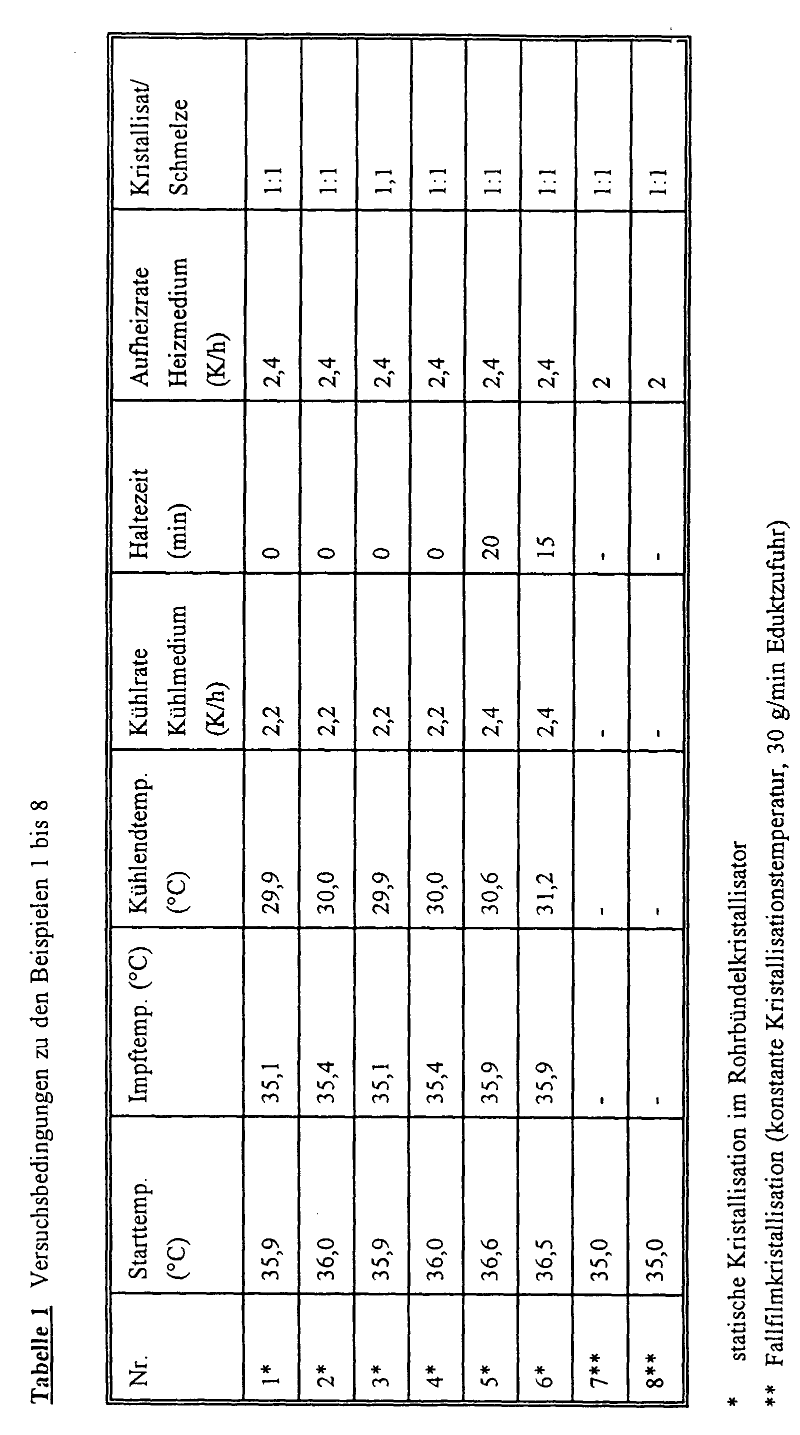

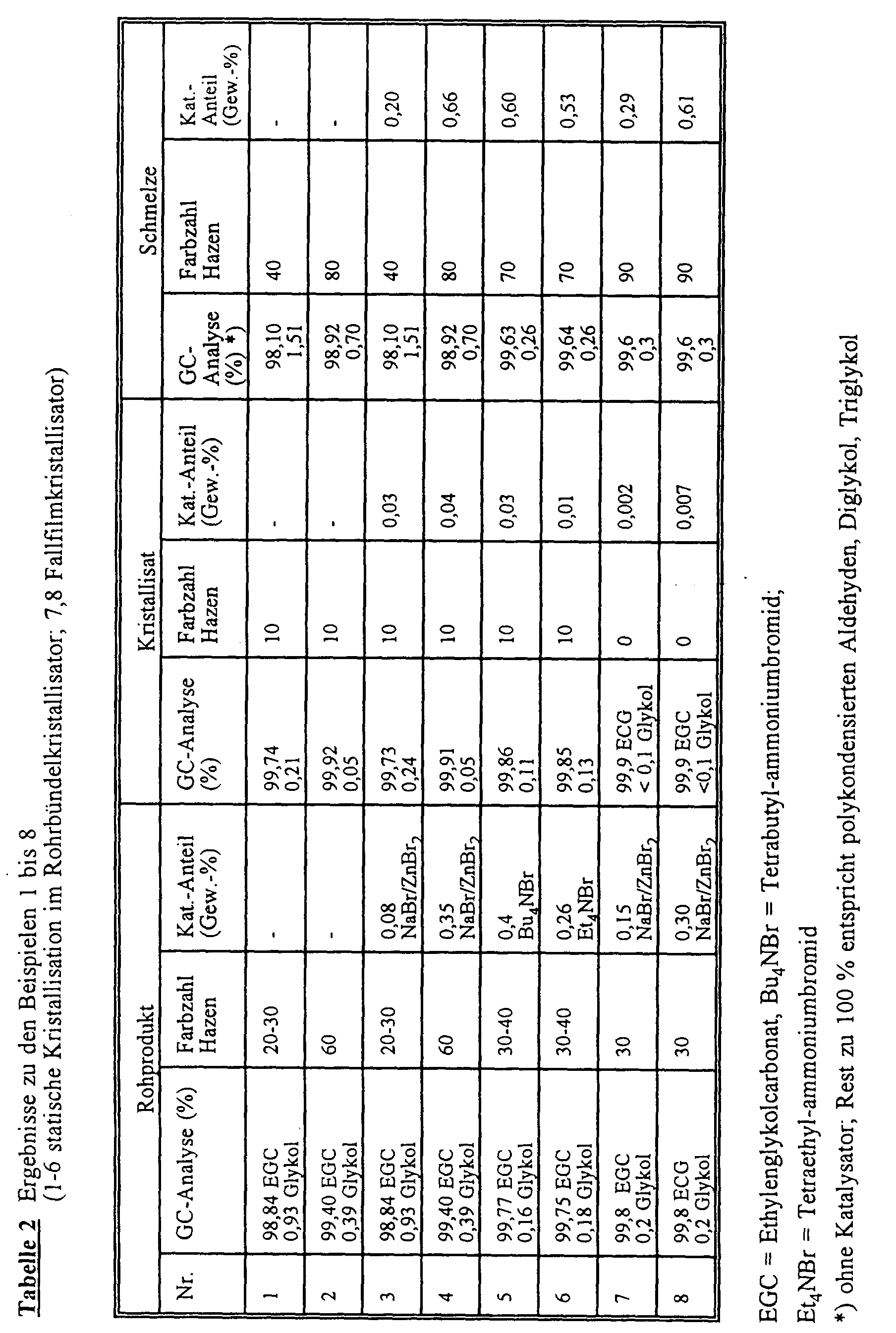

## Patentansprüche

1. Verfahren zur Gewinnung von gereinigtem Ethylenglykolcarbonat (EGC) aus einem verunreinigten EGC, das Verunreinigungen aus der Gruppe von Ausgangsstoffen, Nebenprodukten und/oder Katalysatoren des Herstellungsverfahrens enthält, dadurch gekennzeichnet, daß das verunreinigte EGC einer fraktionierenden Schmelzkristallisation unterworfen wird und das dabei gebildete Kristallisat des gereinigten EGC mechanisch von den zurückbleibenden, in der Restschmelze gelösten Verunreinigungen abgetrennt wird.

2. Verfahren nach Anspruch 1, dadurch gekennzeichnet, daß die fraktionierende Schmelzkristallisation diskontinuierlich durchgeführt wird.

3. Verfahren nach Anspruch 1, dadurch gekennzeichnet, daß die Schmelzkristallisation mit Hilfe eines Rohrbündel-Kristallisators, Plattenwärmeaustauscher unterschiedlicher Konstruktion oder eines Fallfilmkristallisators durchgeführt wird.

4. Verfahren nach Anspruch 1, dadurch gekennzeichnet, daß die Schmelzkristallisation mehrstufig durchgeführt wird.

5. Verfahren nach Anspruch 1, dadurch gekennzeichnet, daß die Trennmethode der Schmelzkristallisation mit einem destillativen Trennschritt kombiniert wird.

6. Verfahren nach Anspruch 1, dadurch gekennzeichnet, daß die Bildung des Kristallisats durch Kristallkeime eingeleitet wird.

7. Verfahren nach Anspruch 3, dadurch gekennzeichnet, daß die Schmelze des zu reinigenden EGC im Bereich von 38 bis 20°C, bevorzugt im Bereich von 37 bis 25°C mit einer Kühlrate des Kühlmediums von 40 bis 0,1 K/h, bevorzugt 10 bis 0,5 K/h abgekühlt wird.

8. Verfahren nach Anspruch 7, dadurch gekennzeichnet, daß nach einer Haltezeit von 0 bis 100 Minuten, bevorzugt 1 bis 70 Minuten am niedrigsten Temperaturpunkt des Kühlmediums die Restschmelze abgetrennt wird und das Kristallisat durch Schwitzen mit einer Heizrate des Heizmediums von 40 bis 0,1 K/h, bevorzugt 20 bis 0,5 K/h, bis zu einer Temperatur von 22 bis 37°C, bevorzugt 28 bis 36°C weiter gereinigt wird.

## Claims

1. Process for the isolation of purified ethylene glycol carbonate (EGC) from a contaminated EGC which contains impurities from the group comprising starting materials, by-products and/or catalysts of the preparation process, characterized in that the contaminated EGC is subjected to a fractional melt crystallization and the crystals of the purified EGC formed in this case are mechanically separated off from the remaining impurities dissolved in the residual melt.

2. Process according to Claim 1, characterized in that the fractional melt crystallization is carried out discontinuously.

3. Process according to Claim 1, characterized in that the melt crystallization is carried out with the aid of a tube-bundle crystallizer, plate heat exchanger of diverse construction or a falling-film crystallizer.

4. Process according to Claim 1, characterized in that the melt crystallization is carried out in multiple stages.

5. Process according to Claim 1, characterized in that the separation method of the melt crystallization is combined with a separation step by distillation.

6. Process according to Claim 1, characterized in that the formation of the crystals is initiated by seed crystals.

7. Process according to Claim 3, characterized in that the melt of the EGC to be purified is cooled in the range from 38 to 20°C, preferably in the range from 37 to 25°C, at a cooling rate of the cooling medium of 40 to 0.1 K/h, preferably 10 to 0.5 K/h.

8. Process according to Claim 7, characterized in that, after a holding time of 0 to 100 minutes, preferably 1 to 70 minutes, at the lowest temperature point of the cooling medium, the residual melt is separated off and the crystals are further purified by sweating at a heating rate of the heating medium of 40 to 0.1 K/h, preferably 20 to 0.5 K/h, up to a temperature of 22 to 37°C, preferably 28 to 36°C.

## Revendications

1. Procédé pour l'obtention d'un carbonate d'éthylène-glycol (EGC) purifié à partir d'un EGC contaminé dont les impuretés proviennent du groupe comprenant des substances de départ, des sous-produits et/ou des catalyseurs du procédé de préparation, caractérisé en ce qu'on soumet le EGC contaminé à une cristallisation fractionnée de la masse fondue et on sépare par voie mécanique le cristallisat du EGC purifié obtenu en l'occurrence des impuretés qui subsistent, dissoutes dans la masse fondue résiduelle.

2. Procédé selon la revendication 1, caractérisé en ce qu'on effectue la cristallisation fractionnée de la masse fondue en discontinu.

3. Procédé selon la revendication 1, caractérisé en ce qu'on effectue la cristallisation de la masse fondue à l'aide d'un dispositif de cristallisation à faisceaux tubulaires, d'un échangeur de chaleur à plateaux de construction diverse ou encore d'un dispositif de cristallisation à film tombant.

4. Procédé selon la revendication 1, caractérisé en ce qu'on effectue la cristallisation de la masse fondue en plusieurs étapes.

5. Procédé selon la revendication 1, caractérisé en ce qu'on combine le procédé de séparation de la cristallisation de la masse fondue avec une étape de séparation par distillation.

6. Procédé selon la revendication 1, caractérisé en ce qu'on amorce la formation du cristallisat via des germes cristallins.

7. Procédé selon la revendication 3, caractérisé en ce qu'on refroidit la masse fondue du EGC à purifier dans le domaine de 38 à 20°C, de préférence dans le domaine de 37 à 25°C avec un débit de refroidissement du milieu réfrigérant de 40 à 0,1 K/h, de préférence de 10 à 0,5 K/h.

8. Procédé selon la revendication 7, caractérisé en ce que, après un temps d'égalisation de 0 à 100 minutes, de préférence de 1 à 70 minutes au point de température le plus bas du milieu réfrigérant, on sépare la masse fondue résiduelle et on soumet le cristallisat à une purification ultérieure par exsudation avec un débit de chauffage du milieu chauffant de 40 à 0,1 K/h, de préférence de 20 à 0,5 K/h jusqu'à une température de 22 à 37°C, de préférence de 28 à 36°C.
